# EUROPEAN PATENT SPECIFICATION

(11) **EP 0 620 231 B1**
(45) Date of publication and mention of the grant of the patent: **20.11.1996**
(21) Application number: 94302422.4
(22) Date of filing: 06.04.1994
(51) Int. Cl.: C07K 14/015, G01N 33/569

(54) **Human parvovirus B19 epitope-related peptide**
Peptid entsprechend einem Epitop des menschlichen Parvovirus B19
Peptide se rapportant à un épitope de parvovirus B19 humain

(30) Priority: 06.04.1993 JP 79802/93
(43) Date of publication of application: 19.10.1994
(73) Proprietor: Shin-Etsu Chemical Co., Ltd., Chiyoda-ku Tokyo 100 (JP)
(72) Inventor: Koumoto, Yasuyoshi, Corp.Res.Center, Takatsu-ku, Kawasaki-shi, Kanagawa 213 (JP); Yahata, Ken, Corp.Res.Center, Takatsu-ku, Kawasaki-shi, Kanagawa 213 (JP); Chiba, Tohru, Corp.Res.Center, Takatsu-ku, Kawasaki-shi, Kanagawa 213 (JP); Nunoue, Tadasu,, Fukuoka-shi, Fukuoka 810 (JP)
(74) Representative: Bassett, Richard Simon

(56) References cited:
- DE-A- 3 939 470
- JOURNAL OF VIROLOGY vol. 65, no. 4 , April 1991 pages 1667 - 1672 HIROYUKI SATO ET AL. 'Identification of the region including the epitope for a monoclonal antibody which can neutralize human parvovirus B19'
- JOURNAL OF VIROLOGY vol. 65, no. 10 , October 1991 pages 5485 - 5490 HIROYUKI SATO ET AL. 'Identification and mapping of neutralizong epitopes of human parvovirus B19 by using human antibodies'

## Description

The present invention relates to epitope peptides useful for specifically detecting antibodies against human parvovirus (HPV) B19, agents for determining the antibodies against HPV B19 containing the epitope peptides and a method for determining the antibodies against HPV B19.

HPV B19 was discovered by Cossart et al. in 1975, in the form of unknown particles present in some human blood samples and it was proved that HPV B19 was a causal virus for man after 1981. The HPV B19 is a causative virus of erythema infectiosum and arthritis in man. For this reason, accurate and correct detection of HPV B19 is required for diagnosis of these diseases. Methods for detecting HPV B19 include, for instance, those which comprise direct detection of the virus or detection of the antibody against the virus. These methods require the use of a substance serving as the antigen for the antibody and this is quite important for the development of such a diagnostic method. Conventionally, there have been used, for instance, HPV B19 originated from human erythroblasts and viral antigenic proteins prepared through gene engineering techniques, as antigenic substances of this kind.

It has generally been believed that an antibody recognizes several amino acid residues on a protein serving as the antigen therefor and the corresponding site is called "epitope". An antigenic protein has several epitopes and, therefore, the virus can easily, specifically be detected or determined without using the viral particles per se if the diagnostic method makes use of a peptide including such a site or epitope.

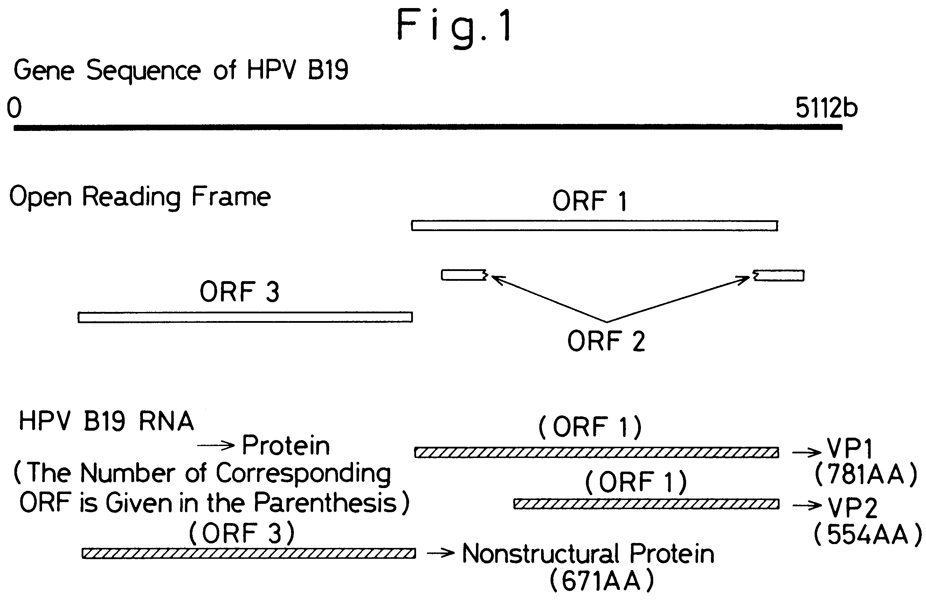

Techniques for solid phase synthesis have recently been advanced and accordingly, various peptides have relatively easily been prepared. Shade et al. elucidated the genome sequence (gene sequence) of HPV B19 which is shown in Fig. 1. Fridell et al. synthesized peptides corresponding to partial regions of open reading frame (ORF) 1 and ORF 2 and found that peptides each corresponding to amino acid residues extending from 236th to 253th amino acids, 284th to 307th amino acids or 732th to 741th amino acids in the ORF 1 region or 161th to 170th amino acids in the ORF 2 region exhibited reactivity with an anti-HPV B19 antibody. Moreover, Sato et al. suggested that a peptide corresponding to the amino acid sequence extending from 328th to 344th amino acids in the viral protein (VP) 2 region reacted with HPV B19.

In these techniques, however, the HPV B19 originated from human erythroblasts is used as such an antigenic substance.

Therefore, they suffer from problems in that it is difficult to obtain such an antigenic substance and that the production thereof through the gene engineering technique requires very complicated procedures. For this reason, there has been desired for the development of a technique which permits the stable and continuous supply of an antigenic substance of this type.

Moreover, any conventional report which examined the reactivity, with HPV B19, of peptides each corresponding to an amino acid sequence assumed to be an antigenic site to the antibody does not perform sufficient fractional quantification of immunoglobulin G (IgG) and immunoglobulin M (IgM). Further, it has not yet been investigated, in detail, whether, or not, the reactivity of the antibody against HPV B19 with a peptide having an amino acid sequence assumed to be an antigenic site of the virus may vary depending on the time elapsed after the viral infection. In addition, the problem of measurement sensitivity has not yet been completely solved.

It is an object of the present invention to provide peptides capable of specifically reacting with antibodies against HPV B19 and to provide applications of the peptides, i.e., agents for determining anti-HPV B19 antibodies and to provide a method for detecting the presence of anti-HPV B19 antibodies, which allows the solution of the foregoing problems associated with the conventional techniques.

The peptide according to the present invention can specifically react with anti-HPV B19 antibodies. For this reason, the peptide can be used as an agent for determining the amount of the anti-HPV B19 antibodies present in a sample such as human serum or plasma. At the same time, the peptide permits the specific detection of the presence of the anti-HPV B19 antibody in such a sample at high sensitivity. Moreover, the peptide of the present invention may likewise be used as an ingredient of a vaccine for treating or preventing erythema infectiosum and arthritis in man caused by HPV B19. Furthermore, the antibodies obtained through the use of the peptide may be used as an agent for treating diseases caused by the virus.

Fig. 1 is a diagram showing the correspondence of the sequence of the gene of HPV B19 with the open reading frame thereof; and the correspondence of the HPV RNA with the HPV B19 protein.

Fig. 2 is a diagram showing the hydrophobicity of the peptide of the present invention determined in terms of the statistical scale of Rose et al.

Fig. 3 is a diagram showing the secondary structure of the peptide of the present invention estimated according to the method of Holley et al.

Fig. 4 is a graph on which results of immunoglobulin G determination are plotted.

Fig. 5 is a graph on which results of immunoglobulin M determination are plotted.

Fig. 6 is a graph on which results of immunoglobulin G determination are plotted, the determination being performed using the biotin-avidin affinity.

The inventors of this invention have made a through investigation of epitope portions reactive with the anti-HPV B19 antibody by synthesizing peptides which extend over the whole region of the viral protein VP2 and carrying out overall simultaneous screening, using Pin Technology Epitope Scanning System (available from Cambridge Research Biochemicals, Inc.).

First of all, the VP2 region (comprising 554 amino acid residues) corresponding to ORF 1 was divided into fractions each comprising 15 residues (wherein 5 residues in each residue belong to the neighboring two fractions) and thus 64 kinds of peptides were synthesized on polyethylene pins. The pins used in the synthesis were surface-coated with acrylic acid and carry a hexamethylenediamine residue as a linker and a β-alanine residue serving as a spacer at the end thereof. The system can satisfy such a requirement that the pin blocks per se formed after the completion of the synthesis can be used on a commercially available microtiter plate for the ELISA assay provided with 96 wells.

The synthesis was initiated by bonding a C-terminal amino acid derivative required for the preparation of an intended peptide to the amino group on the β-alanine residue serving as a spacer. Thereafter, Fmoc amino acid derivatives each was stepwise condensed thereto according to the Fmoc solid phase synthetic method to give protected peptide linked to the pin. The protected peptide thus synthesized was treated in such a manner that only the side group-protecting groups on the amino acid derivatives which constituted the peptide were selectively removed without cutting the synthesized and protected peptide apart from the pin to give a peptide linked to the pin. The selective removal was carried out using TFA/anisole. The 64 kinds of these pin-linked peptides synthesized above were simultaneously inspected for the reactivity with the anti-HPV B19 antibody.

The following Table 1 is prepared on the basis of the foregoing overall simultaneous screening and as a result, some peptides corresponding to sites which specifically react with the anti-HPV B19 antibody were discovered among others.

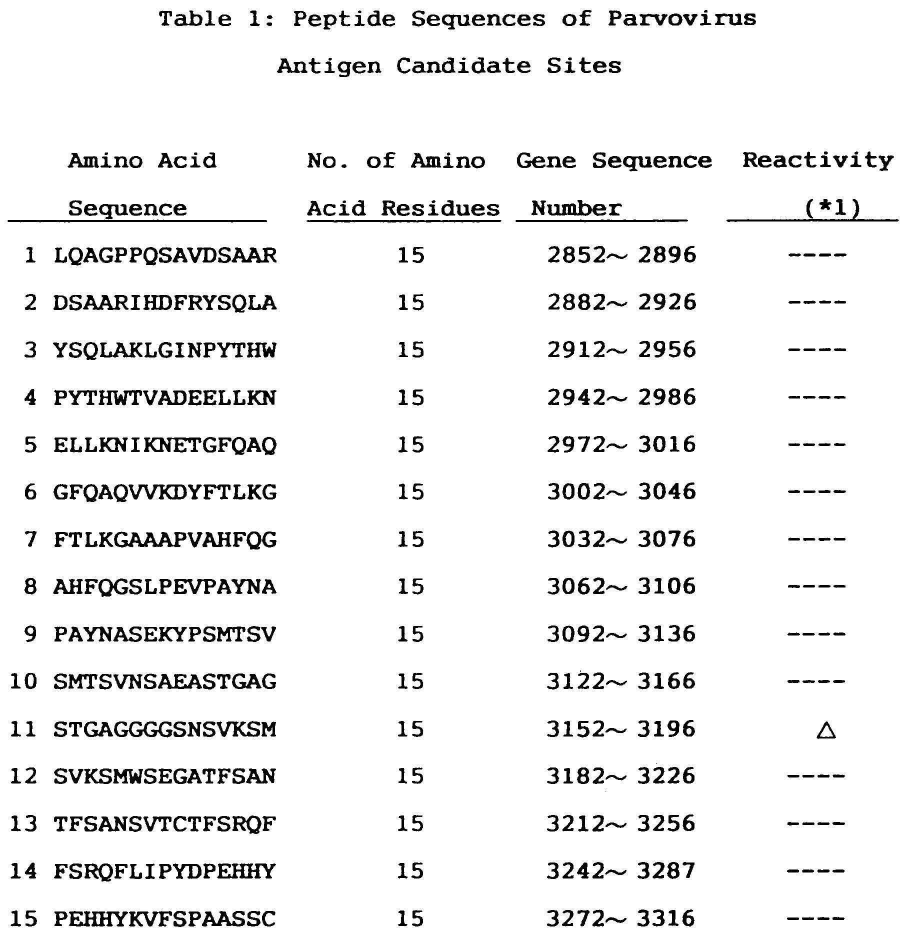

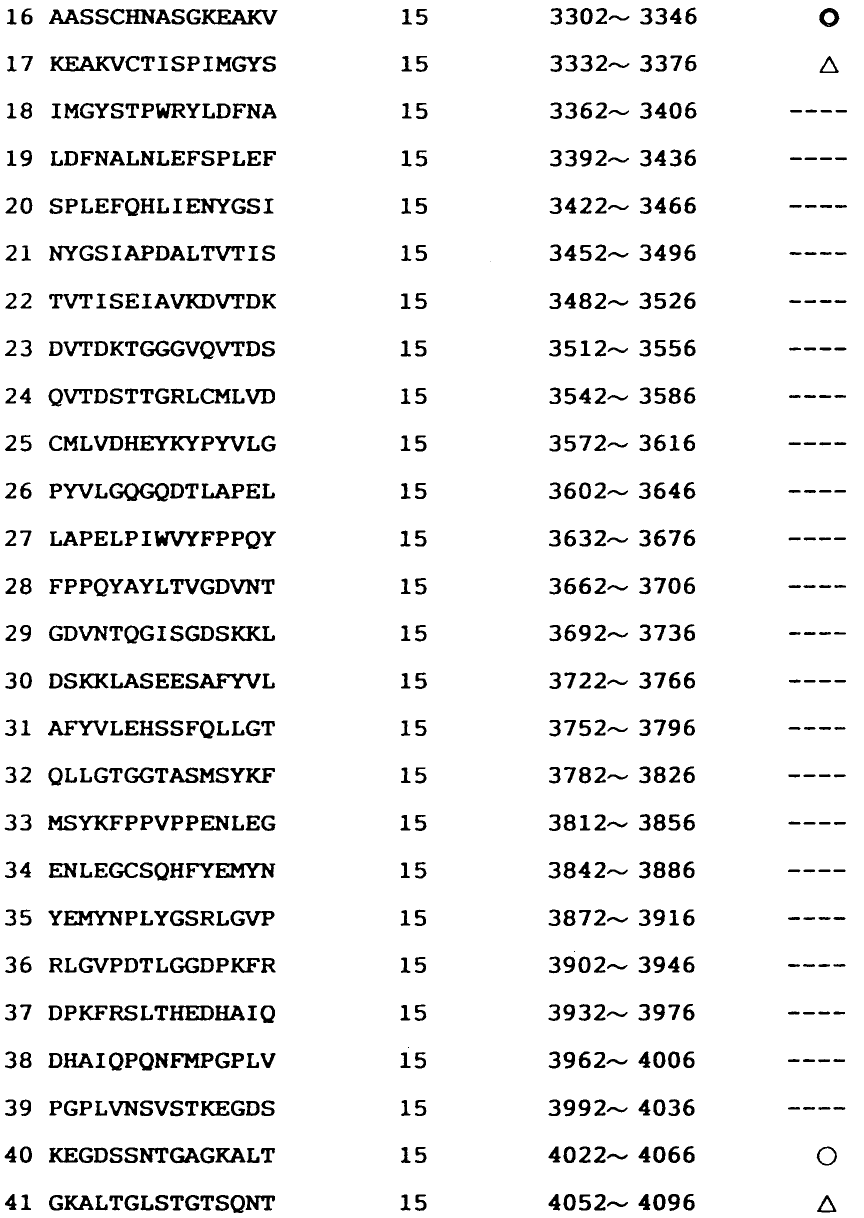

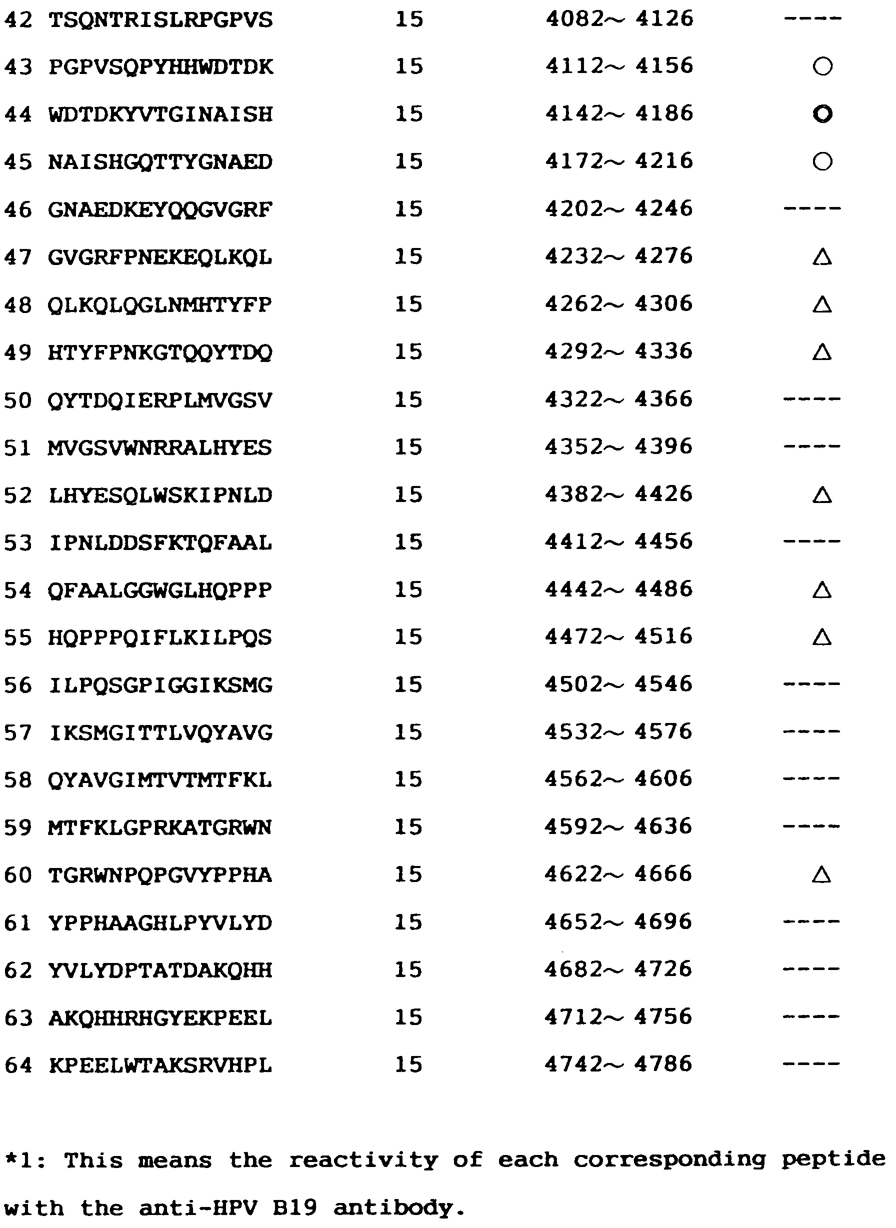

In the VP2 region (comprising 554 amino acid residues) corresponding to ORF 1 of the HPV B19 gene, the amino acid sequence comprising 315 amino acid residues except for 239 amino acid residues lying on the N-terminal side thereof was then subjected to estimation of (i) the hydrophobicity and (ii) the secondary structure while making use of a computer program.

### (i) Hydrophobicity

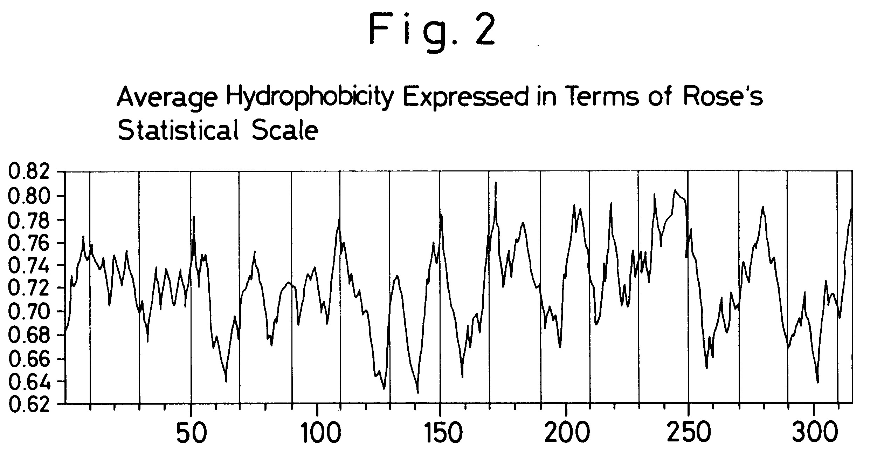

The hydrophobicity of the peptide was determined by plotting average values of every 7 amino acid residues according to the statistical scale of Rose et al. and thus the result as shown in Fig. 2 was obtained, which was an average hydrophobicity expressed in terms of the Rose's statistical scale.

### (ii) Secondary Structure

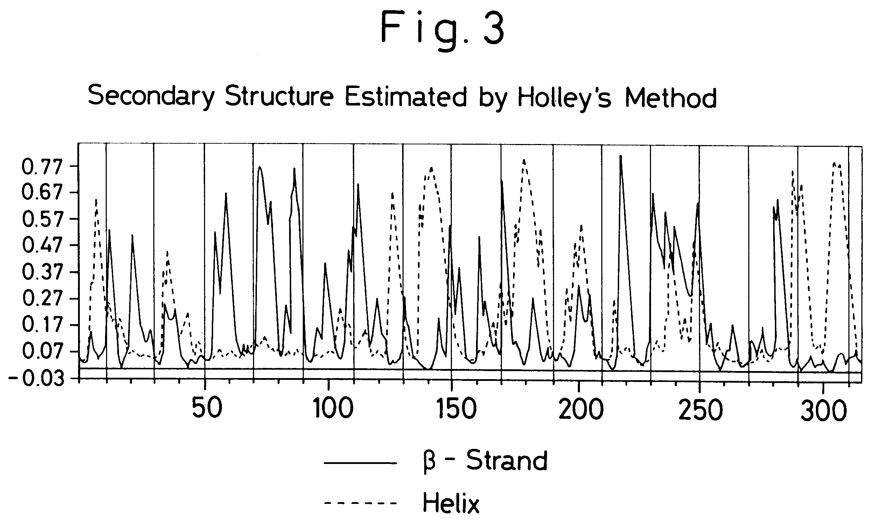

Estimation of the secondary structures of these peptides carried out by the method of Holley et al. provided the conceptional drawing of the secondary structure as shown in Fig. 3.

The synthetical consideration of the results obtained through the overall simultaneous screening and the results of hydrophobicity-determination and secondary structure-determination carried out above leads to discovery of sites which would have antigenicity and the inventors of this invention have narrowed down to several sites.

A secondary screening would be carried out to make clear the reactivity of a free peptide with the anti-HPV B19 antibodies. In this respect, the synthesis of such a free peptide should be performed while taking, into consideration, the degree of difficulty in synthesizing and purifying the corresponding amino acid sequence. Moreover, the number of amino acid residues in the free peptide should be determined after inquiring into possible structures of each amino acid sequence, i.e., the result of the secondary structure-determination using the computer program.

The foregoing primary screening was performed by preparing peptides each equally having 15 amino acid residues therein, but the secondary screening was carried out while preparing various peptides having variously changed amino acid residue numbers due to the reasons discussed above and the inventors have narrowed down to 6 amino acid sequences listed in the following Table 2.

Finally, 6 kinds of free peptides corresponding to the foregoing 6 amino acid sequences were synthesized and were inspected for the reactivity with the anti-HPV B19 antibodies. As a result, there have been discovered, as shown in Table 2, peptides which have amino acid sequences different from the conventionally reported amino acid sequences of peptides, which have high reactivity with the anti-HPV B19 antibodies as compared with the conventionally known peptides and which are peptides corresponding to sites selectively reactive with the antibodies, in particular, at the initial stage (acute stage) of the viral infection.

**Table 2**

| 6 Kinds of Amino Acid Sequences and Reactivity Thereof with Anti-HPV B19 Antibodies | | | |
|---|---|---|---|
| Amino Acid Sequence | Gene Sequence Number | VP2 Amino Acid Sequence No. | Pin No. Corr. to Table 1 |
| STKEGDSSNTGAGKALTGLSTG | 4016∼4081 | 298∼319 | 39, 40, 41 |
| LRPGPVSQPYHHWDTDK | 4105∼4155 | 328∼344 | 42, 43, 44 |
| QPYHHWDTDKYVTGINAISHGQTTY | 4127∼4201 | 335∼359 | 43, 44, 45 |
| WDTDKYVTGINAISHG | 4142∼4189 | 340∼355 | 44, 45 |
| SKIPNLDDSFKTQFAALGGWGL | 4405∼4470 | 428∼449 | 52, 53, 54 |
| QFAALGGWGLHQPPPQIF | 4442∼4494 | 440∼457 | 53, 54, 55 |

| Amino Acid Sequence | Average-Hydrophobicity (Rose) | Secondary Structure (Holley) | Reactivity with Anti-HPV B19 Antibodies |
|---|---|---|---|
| STKEGDSSNTGAGKALTGLSTG | ○ | β-strand | ○ |
| LRPGPVSQPYHHWDTDK | ○∼△ | β | ○ |
| QPYHHWDTDKYVTGINAISHGQTTY | ○ | β∼α∼β | ⓞ |
| WDTDKYVTGINAISHG | ○ | α∼β | ○ |
| SKIPNLDDSFKTQFAALGGWGL | △∼○ | α | X |
| QFAALGGWGLHQPPPQIF | ○ | α | X |

Accordingly, it has been found out that the peptide highly reactive with the anti-HPV B19 antibodies has the amino acid sequence represented by the following formula (1):
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH (1)

The peptide having a series of amino acid residues represented by the foregoing formula (1) corresponds to the site (extending from 335th amino acid residue to 359th amino acid residue) in the ORF 1 region shown in Fig. 1 which is a diagram showing the correspondence of the sequence of the gene of HPV B19 with the open reading frame thereof; and the correspondence of the HPV RNA with the HPV B19 protein.

This peptide represented by Formula (1) and the aforementioned peptide in the VP2 region extending from 328th amino acid residue to 344th amino acid residue reported by Sato et al. have 10 common amino acid residues (or 10 amino acid residues are overlapped between these two peptides), but the reactivity of the peptide of Formula (1) with the anti-HPV B19 antibodies is extremely high as compared with the peptide corresponding to the site reported by Sato et al. and the former is characterized in that it exhibits high reactivity therewith, in particular, in the initial stage of the viral infection. Therefore, the peptide can provide a diagnostic method which exhibits high measurement sensitivity and which can be used depending on the lapse of time after the infection or the crisis of the diseases caused by HPV B19.

Examples of methods for preparing the intended peptide include enzymatic decomposition of proteins and chemical synthetic methods, with the chemical synthetic method being preferably used in the present invention because of their easiness and ability of mass production of the intended peptide. The chemical synthetic method may be liquid phase or solid phase one and the solid phase method may be t-butoxycarbonyl (Boc) method or 9-fluorenyl-methoxycarbonyl (Fmoc) method. When a solid phase method is adopted, automatic synthesizer may be used. In these peptides prepared according to these chemical methods, the functional side group of each amino acid constituting the peptides is protected and accordingly, the protective group should be ultimately removed. More specifically, these protective groups are removed from the amino acid moieties by any method commonly employed in this field such as a hydrogen fluoride methods, catalytic reduction methods, trifluoroacetic acid methods, bromotrimethylsilane (TMSBr) methods or trimethylsilyl trifluoromethanesulfonate (TMSOTf) methods to give the intended peptide. It is desirable to purify the peptide thus obtained through the reverse phase liquid chromatography technique at the final stage.

The peptide of Formula (1) has specificity and high affinity to the anti-HPV B19 antibodies and accordingly, can be used as agents for detecting the anti-HPV B19 antibodies at high sensitivity.

In addition, the anti-HPV B19 antibodies can be detected, through the use of the foregoing peptide of Formula (1) by any known immunological method. Specific examples of such immunological methods include the radioimmunoassay (RIA) technique, the enzyme-linked immunosorbent assay (ELISA) technique, the fluoroimmunoassay (FIA) technique, the chemiluminescent immunological method and the immuno-agglutination technique.

Elements for each measurement system of this type comprise the immobilized solid support, the sample to be examined and the labeled antibody or the labeled antigen. The term "immobilized solid support" herein used means the solid support such as glass, plastic or polymeric substance, bound with the substance capable of binding with the sample to be examined. The solid support may have a variety of shapes such as test tube-like, microtiter plate-like, cuvette-like, beads-like, fine particle-like and membrane-like ones. The term "substance capable of binding with the sample to be examined" is herein defined to be the substance having an ability of binding with antibodies, such as the antigen or against the sample to be examined, antibodies protein A. The solid support bound with the foregoing substance through, for instance, physical adsorption method or covalent-bonding method. Alternatively, the solid support may be bound with the substance capable of binding with the sample through substances having any affinities available such as antibody/anti-antibody affinity, biotin/avidin affinity, protein A and protein G/antibody affinity and lectin/carbohydrate affinity.

The immobilized solid support is desirably treated with a blocking agent to deactivate remaining sites and to thus suppress the occurrence of any non-specific adsorption reaction at the remaining sites on the surface of the support. The term "blocking agent" herein used means a substance which does not take part in any specific reaction in the detection system and specific examples thereof include natural and synthetic polymeric materials such as bovine serum albumin, ovalbumin, components of milk, protein components such as protein-decomposition products, gelatin and polyvinyl alcohol. The sample to be examined may be any ones so far as they comprise antibodies, for instance, sera and plasma. Among the foregoing elements of the measurement system, the labeled antibody or antigen may comprise an antigen or antibody and a labeling substance. These antigen or antibody and the labeling substance may be bound through the aforementioned substances exhibiting affinity. The sample to be examined may likewise be bound with the labeled antibody or antigen through a substance having affinity. The peptide according to the present invention may be used, in the foregoing measurement system, as the immobilized solid support or the labeled antigen which is one of the elements of the system.

The synthesis of the peptide of the present invention and the determination of the reactivity of the peptide with the anti-HPV B19 antibody will be detailed below.

### Peptide Preparation Example

The peptide was prepared according to the solid phase synthetic method. More specifically, a protective peptide was prepared by the Fmoc solid phase method using 430A Synthesizer available from Applied Biosystem Inc. Protective groups of the resulting protective peptide were removed therefrom according to the TMSOTf method to give a crude peptide. The crude peptide was purified by, in order, gel filtration and reverse phase liquid chromatography to give a final purified peptide represented by the following formula (1):
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH (1)

There were prepared and used, by way of comparison, a peptide represented by the following formula (2) (reported by Fridell et al.):
H-Phe-Ser-Pro-Ala-Ala-Ser-Ser-Cys-His-Asn-Ala-Ser-Gly-Lys-Glu-Ala-Lys-Val-Cys-Thr-Ile-Ser-Pro-Ile-OH (2)
and a peptide represented by the following formula (3) (reported by Sato et al.):
H-Leu-Arg-Pro-Gly-Pro-Val-Ser-Gln-Pro-Tyr-His-His-Trp-Asp-Thr-Asp-Lys-OH (3)

### Detection Example 1: Detection of Immunoglobulin G (IgG)

In this example, the serum obtained from a patient infected with HPV B19 was inspected for the presence of an IgG antibody by the ELISA method. A microtiter plate made of polystyrene was used as a solid support. A peptide solution whose peptide concentration was adjusted to 10µg/mℓ with a phosphate buffer was dispensed to the wells (100µℓ each) of the plate and then the peptide was allowed to be immobilized at room temperature overnight to give an immobilized solid support. The immobilized solid support was then treated with blocking agent for inhibiting any non-specific adsorption reaction of unlinked sites present on the solid support with the IgG antibody. Then the serum obtained from a patient was diluted 200 times with a commercially available diluent, the diluted serum was dispensed to the wells (100 µℓ each) and they were reacted at 37 °C for 2 hours. The serum used in the experiment was collected from a 12-year-old female child who had an aplastic crisis due to infection with HPV B19.

The microtiter plate was washed 6 times with a phosphate buffer, then horseradish peroxidase (HRP)-labeled anti-human IgG antibody which was diluted 1000 times with the same buffer was dispensed to the wells (100µℓ each) and they were reacted at 37°C for one hour. Thereafter, the reaction system was further washed 6 times with a phosphate buffer, o-phenylenediamine (OPD: OPD 4 mg/10 mℓ H₂O, H₂O₂ 15µℓ) as a color-developing agent was dispensed to the wells (100µℓ each), followed by reaction at room temperature for 30 minutes and termination of the reaction through addition of 100 µℓ each of 1N H₂SO₄. The colored product was inspected for the presence of HRP through determination of absorbance at a wavelength of 492 nm using a microtiter plate reader.

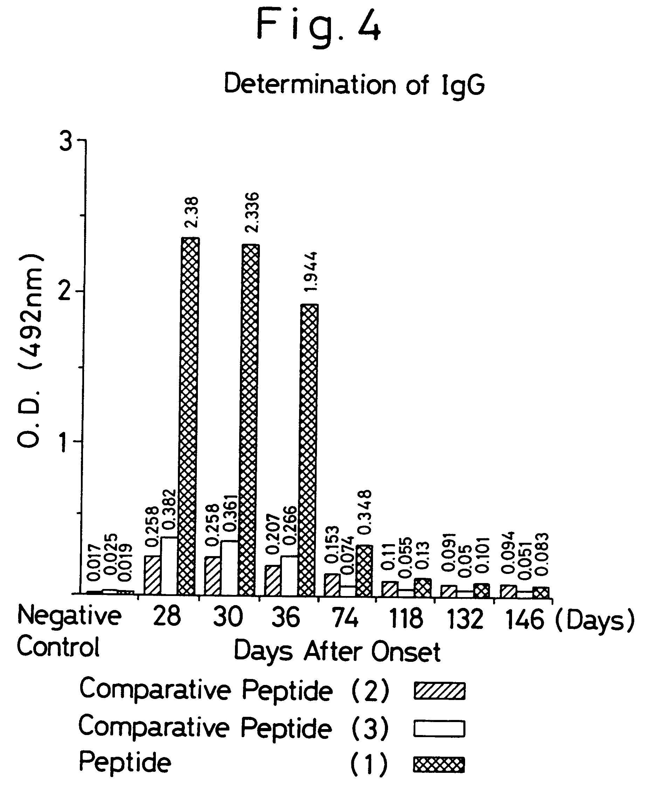

The peptide of Formula (1) was used in this determination. In addition, the peptides of Formulae (2) and (3) were also used as comparative peptides. The results thus obtained are plotted on Fig. 4. In Fig. 4, the time after onset of illness is plotted as abscissa and the absorbance as ordinate. The results shown in Fig. 4 clearly indicate that the peptide of Formula (1) exhibits high reactivity with the anti-HPV B19 antibody (IgG). Moreover, it also becomes clear that the peptide shows strong reactivity at the initial stage of the infection (the acute stage).

### Detection Example 2: Detection of Immunoglobulin M (IgM)

In this example, the serum obtained from a patient infected with HPV B19 was inspected for the presence of an IgM antibody by the ELISA method. The same procedures used in Detection Example 1 were repeated to give an immobilized solid support and to carry out the same assay except that HRP-labeled human IgM antibody was substituted for the HRP-labeled human IgG antibody used in Detection Example 1.

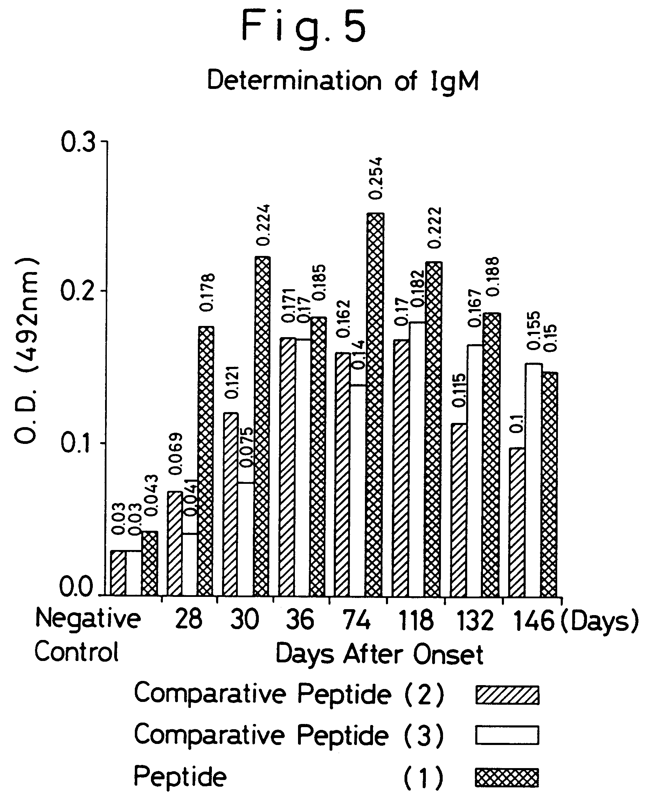

The peptide of Formula (1) was used in this determination. In addition, the peptides of Formulae (2) and (3) were also used as comparative peptides. The results thus obtained are plotted on Fig. 5. The results shown in Fig. 5 clearly indicate that the peptide of Formula (1) also exhibits high reactivity with IgM among the anti-HPV antibodies.

### Detection Example 3: Determination of IgG While Making Use of Biotin-Avidin Binding Ability

This Example is given for illustrating a method in which the immobilized solid support is prepared by immobilizing peptide onto the solid support through the use of the biotin-avidin affinity to thus determine the IgG antibody. First of all, a 250 µg/mℓ solution of biotin-N-hydroxysuccinimide ester (NHS-Biotin) was dispensed to wells (100µℓ each) of a microtiter plate of polystyrene carrying, on the surface, amino groups and the reaction was continued at 37 °C for 2 hours. Then the microtiter plate was washed 6 times with a phosphate buffer, a 50µg/mℓ solution of avidin was dispensed to the wells (100µℓ each) and they were reacted at 37 °C for one hour.

The biotinylated peptide used herein was obtained by coupling NHS-Biotin to the N-terminal of the protected peptide prepared in the same manner used in the foregoing Peptide Preparation Example followed by removal of the protective groups and purification carried out according to the same manner used in the foregoing Peptide Preparation Example. The resulting biotinylated peptide was washed 6 times with a phosphate buffer and subjected to blocking and assay according to the same method used in Detection Example 2.

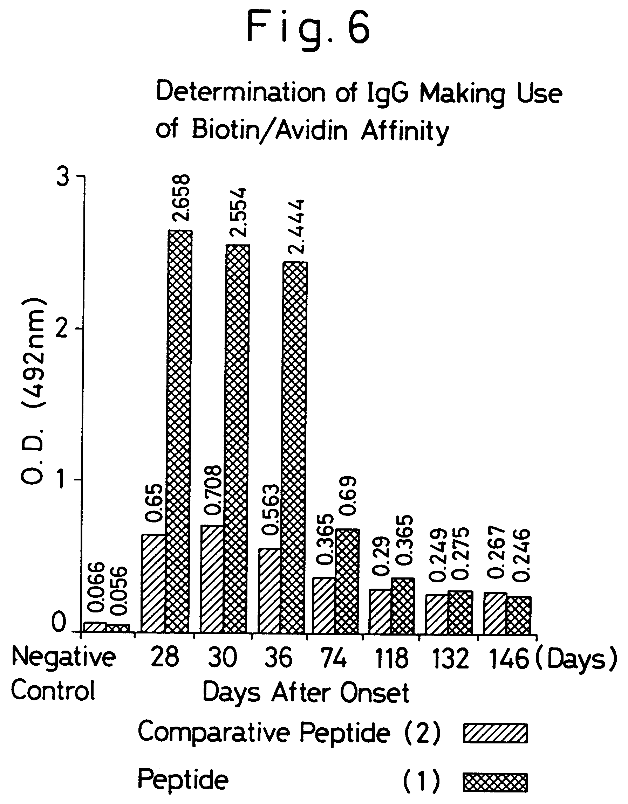

The peptide of Formula (1) was used in this determination. In addition, the peptides of Formulae (2) and (3) were also used as comparative peptides. The results thus obtained are plotted on Fig. 6. The results shown in Fig. 6 clearly indicate that the peptide of Formula (1) exhibits high reactivity with the anti-HPV B19 antibodies. Moreover, it also becomes clear that the peptide shows strong reactivity at the initial stage of the infection (the acute stage). Incidentally, amino acids are herein denoted according to the recommendation of the Committee on Biochemical Nomenclature of IUPAC and IUB. Moreover, the amino acids used herein are L-type ones unless otherwise specified.
- alanine: : Ala
- arginine: : Arg
- asparagine: : Asn
- aspartic acid: : Asp
- cysteine: : Cys
- glutamine: : Gln
- glutamic acid: : Glu
- glucine: : Gly
- histidine: : His
- isoleucine: : Ile
- leucine: : Leu
- lysine: : Lys
- methionine: : Met
- phenylalanine: : Phe
- proline: : Pro
- serine: : Ser
- threonine: : Thr
- tryptophan: : Trp
- tyrosine: : Tyr
- valine: : Val

## Claims

1. A peptide having an amino acid sequence represented by the following formula:
H-Gln-Pro-Tyr-His-His-Trp-Asp-Thr-Asp-Lys-Tyr-Val-Thr-Gly-
Ile-Asn-Ala-Ile-Ser-His-Gly-Gln-Thr-Thr-Tyr-OH
and reactivity with an antibody against human parvovirus B19.

2. An agent for inspecting the presence of an antibody against human parvovirus B19 having an amino acid sequence represented by the following formula:
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH

3. A method for detecting human parvovirus B19 comprising the step of subjecting an antibody against human parvovirus B19 to a reaction with a peptide having an amino acid sequence represented by the following formula:
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH

## Patentansprüche

1. Peptid mit einer Aminosäuresequenz entsprechend der folgenden Formel:
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH
und Reaktionsfähigkeit gegenüber einem Antikörper gegen Human-Parvovirus B19.

2. Mittel zur Überprüfung des Vorhandenseins eines Antikörpers gegen Human-Parvovirus B19 mit einer Aminosäuresequenz der folgenden Formel:
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH

3. Verfahren zum Nachweis von Human-Parvovirus B19 durch Reagierenlassen eines Antikörpers gegen Human-Parvovirus B19 mit einem Peptid mit einer Aminosäuresequenz der folgenden Formel:
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH

## Revendications

1. Peptide possédant une séquence d'aminoacides représentée par la formule suivante :
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH
et présentant une réactivité avec un anticorps agissant contre un parvovirus humain B19.

2. Agent pour rechercher la présence d'un anticorps agissant contre un parvovirus humain B19 possédant une séquence d'aminoacides représentée par la formule suivante :
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH

3. Procédé de détection d'un parvovirus humain B19 comprenant l'étape consistant à soumettre un anticorps agissant contre un parvovirus humain B19 à une réaction avec un peptide possédant une séquence d'aminoacides représentée par la formule suivante :
H-Gln-Pro-Tyr-His-His-Trp-Asp-Thr-Asp-Lys-Tyr-Val-Thr-Gly-Ile-Asn-Ala-Ile-Ser-His-Gly-Gln-Thr-Thr-Tyr-OH
